(19) Europäisches Patentamt
European Patent Office
Office européen des brevets

(11) Veröffentlichungsnummer: **0 150 796**
**B1**

(12) # EUROPÄISCHE PATENTSCHRIFT

(45) Veröffentlichungstag der Patentschrift: **27.09.89**

(51) Int. Cl.[4]: **C 12 N 5/02**, C 08 B 37/02

(21) Anmeldenummer: **85100611.4**

(22) Anmeldetag: **22.01.85**

(54) **Zellkulturmikroträger, Verfahren zu seiner Herstellung und seine Verwendung zum Züchten von verankerungsabhängigen Zellen.**

(30) Priorität: **28.01.84 DE 3402927**

(43) Veröffentlichungstag der Anmeldung: **07.08.85 Patentblatt 85/32**

(45) Bekanntmachung des Hinweises auf die Patenterteilung: **27.09.89 Patentblatt 89/39**

(84) Benannte Vertragsstaaten: **AT BE CH DE FR GB IT LI LU NL SE**

(56) Entgegenhaltungen:
**EP-A- 0 066 135**
**EP-A- 0 066 726**
**US-A- 3 910 819**

**PRESENTATION FROM BIOTECH 1983, London, 4.-6. Mai 1983, Seiten 47-55, Pharmacia Fine Chem., SE; J. CLARK et al.: "Recent developments in microcarrier cell culture"**

(73) Patentinhaber: **PFEIFER & LANGEN, Linnicher Strasse 48, D-5000 Köln 41 (DE)**

(72) Erfinder: **Schwengers, Dieter, Dr., Jussenhovener Strasse 37, D-4047 Dormagen (DE)**
Erfinder: **Keller, Ingrid, Rochusstrasse 21, D-4050 Mönchengladbach (DE)**

(74) Vertreter: **Werner, Hans-Karsten, Dr. et al, Deichmannhaus am Hauptbahnhof, D-5000 Köln 1 (DE)**

**EP 0 150 796 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der vorliegenden Erfindung sind Zellkulturmikroträger mit positiv geladenen chemischen Anteilen aus vernetzten Polysacchariden und damit verknüpften basischen Gruppen, Verfahren zu ihrer Herstellung und ihre Verwendung zur Züchtung verankerungsabhängiger Zellen in einer Mikroträgerkultur.

Die Grundlagen der Haltung und Vermehrung menschlicher und tierischer Zellen in Zellkulturen wurden während der letzten 20 Jahre systematisch entwickelt. Zellkulturverfahren sind heute in vielen Laboratorien zur Produktion von Impfstoffen, Antikörpern, Interferon, Enzymen und Hormonen fest etabliert.

Primärzellen und diploide Zellen benötigen für ihr Wachstum eine feste Unterlage mit einer ausgeprägten Oberflächenladung. Diese Zellen werden als verankerungsabhängige Zellen bezeichnet, da sie nur dann wachsen können, wenn sie sich an einem Träger verankern können.

Als Träger ist ursprünglich vor allem Glas in Form von Petrischalen, Kulturflaschen und Rollkulturflaschen verwendet worden. Auf der Oberfläche der Geräte kann sich jedoch nur eine Monoschicht der Zellen ausbilden, so daß die pro Gerät zur Verfügung stehende Oberfläche begrenzt bleibt. Im industriellen Maßstab müssen daher für derartige Zellvermehrungskulturen Tausende von Rollkulturflaschen gespült, sterilisiert, mit Nährmedium gefüllt, mit Zellen beimpft und nach beendeter Zellvermehrung abgeerntet werden. Da diese Schritte alle unter sterilen Bedingungen ausgeführt werden müssen, sind sie sehr arbeits- und kostenintensiv.

Mit der Einführung der Zellkulturvermehrung auf Mikroträgern durch A. L. von Wezel, Nature 216 (1967) S. 64 wurde eine verbesserte Technologie entwickelt, welche die geschilderten Probleme beseitigen könnte. Bei dieser Technik werden Mikroträger mit einer durchschnittlichen Größe im Bereich von 100 bis 300 µm in einem Nährmedium suspendiert. Sofern die Mikroträger eine geeignete Oberflächenladung aufweisen, heften sich die verankerungsabhängigen Zellen an die Mikroträger an und wachsen auf ihnen. 2 bis 5 g/l (Trokkengewicht) Mikroträger stellen im Nährmedium eine Oberfläche von 0,76 bis 1,9 $m^2$/l Kulturvolumen zur Verfügung. Die größten Rollerflaschen bieten hingegen nur eine Oberfläche von etwa 0,16 $m^2$ an. 1 l Kultur mit Mikroträgern ist daher in der Lage 5 bis 12 große Rollerflaschen zu ersetzen. Mikroträger haben jedoch im industriellen Maßstab bisher noch nicht die Bedeutung erlangt, die man zunächst erwartet hatte, da bisher entwikkelte Mikroträger noch eine Reihe von Nachteilen aufweisen.

A.L. van Wezel verwendete ursprünglich unlösliche Dextranperlen, die mit Diethylaminoethylgruppen substituiert sind und als DEAE-Sephadex A-50 (Pharmacia AB, Schweden) im Handel erhältlich sind. Bei ihrer Verwendung zeigten sich jedoch cytotoxische und Nährmitteladsorptionseffekte, die sich in einem anfänglichen Absterben von Zellen sowie durch ein unbefriedigendes Zellenwachstum äußerten; vgl. C.-B. Horng, W. Melimaus (Biotechnology and Bioengineering Vol. 17 (1975) S. 713–732). Es wurden in der Zwischenzeit verschiedene Verbesserungen für Mikroträger entwickelt, die in der Lage sind einige der ursprünglichen Nachteile zu beseitigen. So wird in der DE-OS 2 909 340 ein Verfahren zur Vorbehandlung von Mikroträgern für Zellkulturen beschrieben, bei welchem die Perlen mit fötalem Kälberserum getränkt und etwa 10 min. in dem Serum auf 75 bis 90 °C erwärmt werden.

Levine, Wong, Wang und Thilly experimentierten mit Mikroträgern verschiedener Ladungsdichte und kamen zu dem Ergebnis, daß DEAE-Dextranperlen mit einem Durchmesser von 150 µm und einer Ladungsdichte von 2 meq/g der trockenen Dextranmatrix für das Verankern von Zellen und ihr Wachstum optimal sind; vgl. D.W. Levine et al in Somatic Cell Genetics Vol. 3 (1977) S. 149–155, US-PS 4 189 534 sowie DE-OS 2 749 989.

Derartige Mikroträger, die anstelle von 5,4 meq/g nur noch 1,5 bis 2,0 meq/g Ladungsdichte aufweisen, wurden inzwischen von der Firma Pharmacia AB unter der Bezeichnung «Cytodex-1» sowie von der Firma Flow Laboratories unter der Bezeichnung «Superbeads» in den Handel gebracht. Einen Überblick über die Eigenschaften und Anwendungsmöglichkeiten von diesen Produkten sowie eine Einführung in die Entwicklung der Mikroträgertechnologie gibt die Firmenschrift «Microcarrier cell culture, Principles + methods» Auflage 1981, erhältlich von der Firma Pharmacia AB, Uppsala, Schweden.

Leider hat sich gezeigt, daß auch diese verbesserten Mikroträger mit einer deutlich verminderten Ladungskapazität in der Praxis vor allem bei empfindlichen Zellen noch immer cytotoxische Effekte aufweisen.

Die EP-OS 66 726 geht davon aus, daß diese cytotoxischen Eigenschaften der Mikroträger auf DEAE-Dextranbasis in der chemischen Struktur der Mikroträger selbst liegen sollen. So ist es bekannt, daß DEAE-Mikroträger neben dem DEAE-Substituenten mit tertiär gebundenem Stickstoff auch Gruppen mit quarternär gebundenem Stickstoff enthalten, die sich bei der Synthese der Mikroträger durch eine nie ganz zu vermeidende erneute Umsetzung mit weiteren Chlorethyldiethyl-amin bilden. Von in diesen sogenannten «Tandem»-Gruppen wird vermutet, daß sie alkylierend wirken und toxisch sind; vgl. L. Ahrgren et al in «Polymeric Amines and Ammonium Salts» E.J. Goethals, Pergamon Press S. 293–294. Auch in der Firmenschrift «Microcarrier cell cultur», Pharmacia AB, S. 27 wird darauf hingewiesen, daß mit den bekannten Herstellungsverfahren für DEAE-Dextran-Mikroträger bis 35% Tandemgruppen gebildet werden. In dem Produkt Cytodex-1 sei der Tandemgruppengehalt auf etwa 15% vermindert.

Um diesen vermuteten cytotoxischen Effekt ganz auszuschließen, werden in der EP-OS 66 726 Mikroträger beschrieben, die nur quarternäre

Aminogruppen enthalten. Derartige Mikroträger sind unter der Bezeichnung «Cytodex-2» von der Pharmacia AB, Schweden in den Handel gebracht worden. Sie weisen eine Ladungskapazität von 0,5 bis 0,8 meq/g auf.

Trotz dieser noch weiter verminderten Ladungskapazität haben auch diese Mikroträger in der Praxis noch nachteilige Effekte gezeigt, die auf ein Absorbieren von Kulturmediumbestandteilen an den Träger zurückgeführt werden. In der DE-OS 3 033 885 werden daher ladungsfreie Mikroträger beschrieben, die mit Polypeptiden wie Kollagen oder Gelatine beschichtet sind. Auf diesen Mikroträgern können sich jedoch nur Zellen verankern, die auf ihrer Oberfläche Strukturelemente enthalten, die eine ausreichende Bindungsaffinität zur Polypeptidschicht auf dem Mikroträger aufweisen.

Die Erfindung hat sich die Aufgabe gestellt, Zellkulturmikroträger mit positiv geladenen chemischen Anteilen aus vernetzten Polysacchariden und damit verknüpften basischen Gruppen zu entwickeln, die alle zuvorgenannten Nachteile nicht aufweisen. Insbesondere hat sich die Erfindung die Aufgabe gestellt, Mikroträger zu entwickeln, die auch im industriellen Maßstab auch zur Züchtung empfindlicher Zellen genutzt werden können und somit praktisch keine Toxizität aufweisen.

Diese Aufgabe kann überraschenderweise gelöst werden, dadurch daß der Zellkulturmikroträger nur mit den positiv geladenen, basischen «Tandem»-Gruppen mit einem pKs-Wert von 5–8 der Formel (I) substituiert ist,

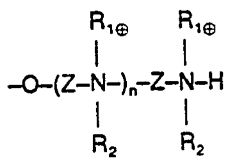 (I)

in welcher Z eine Kohlenwasserstoffkette aus 2–4 Kohlenstoffatomen ist, $R_1$ und $R_2$ gleich oder verschieden sind und Methyl-, Ethyl-, Propyl-, Isopropyl-, Butyl-, Phenyl-, Toluol- oder Benzylgruppen darstellen und n 1 bis 3 ist.

Zellkulturenmikroträger mit derartigen positiv geladenen basischen Gruppen haben sich überraschenderweise als nicht toxisch erwiesen, obwohl die Fachwelt davon ausging, daß gerade diese Gruppen besonders toxisch seien und deshalb nach Möglichkeit nicht oder nur in geringer Zahl an den Zellkulturmikroträgern vorhanden sein sollten. Überraschenderweise dürfen diese positiv geladenen basischen Gruppen auch in hoher Zahl vorhanden sein, so daß es nicht nötig ist, die Anzahl der meq/g trockenen, vernetzten Polysacchariden zu beschränken. Ein Grund für diese überraschend guten Eigenschaften der erfindungsgemäßen Zellkulturmikroträger scheint darin zu liegen, daß es nicht auf die Ladungsdichte ankommt, sondern auf den pKs-Wert der positiv geladenen basischen Gruppen. Während die basischen Gruppen von DEAE-Sephadex bei 9,2 liegen, liegen die pKs-Werte der erfindungsgemäßen Zellkulturmikroträger im Bereich von 5 bis 8. Bei einem erfindungsgemäß bevorzugten und leicht herzustellenden Zellkulturmikroträger, bei welchem Z eine Ethylengruppe und $R_1$ und $R_2$ eine Ethylgruppe darstellen und n gleich 1 ist, beträgt der pKs-Wert 6,2. Ein derartiger Zellkulturmikroträger ist somit praktisch nur mit «Tandem»-Gruppen substituiert.

Außer dem erwähnten einfachsten Fall von «Tandem»-Gruppen, können erfindungsgemäß auch solche Gruppen zur Anwendung kommen, in denen Z 3, 4 oder mehr Kohlenstoffatome aufweist und in denen $R_1$ und $R_2$ neben der Ethylgruppe auch Methylgruppen, Propylgruppen, Isopropylgruppen und Butylgruppen darstellen. Da die pKs-Werte immer noch in dem Bereich von etwa 5 bis 8 liegen, können $R_1$ und $R_2$ auch Arylgruppen wie Phenyl- und Toluolgruppen sowie Arakylgruppen wie insbesondere Benzylgruppen sein. Da die pKs-Werte in dem Bereich von 5 bis 8 liegen, kann n auch größer als 1 sein, so daß auch basische Gruppen aus 3 oder 4 Aminogruppen zur Anwendung kommen können.

Als negative Gegenionen zu den positiv geladenen Stickstoffatomen können prinzipiell alle physiologisch verträglichen anorganischen oder organischen Anionen verwendet werden. Geeignet sind insbesondere Chlorid-, Phosphat-, Sulfat- und Acetationen.

Die pKs-Werte der basischen Substituenten der vernetzten Polysaccharide können in einfacher Weise durch Titration ermittelt werden. Die Titrationskurven weisen am pKs-Wert den bekannten Wendepunkt auf.

Die Erfindung betrifft weiterhin das Verfahren zur Herstellung der neuen Zellkulturmikroträger. Die Herstellung erfolgt durch an sich bekannte Umsetzung der vernetzten Polysaccharide mit Substanzen der Formel II

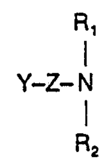 (II)

in der Z eine Kohlenwasserstoffkette aus 2–4 Kohlenstoffatomen ist, $R_1$ und $R_2$ gleich oder verschieden sind und Methyl-, Ethyl-, Propyl-, Isopropyl-, Butyl-, Phenyl-, Toluol-, oder Benzylgruppen darstellen und Y eine reaktive Gruppe ist. Als reaktive Gruppen kommen insbesondere Chlorid, Bromid aber auch Sulfonsäuregruppen etc. in Frage, die in der Lage sind eine O-Alkylierung des vernetzten Polysaccharids durchzuführen.

Die Umsetzung erfolgt erfindungsgemäß in Gegenwart von starken Basen in mindestens 2 Stufen und mindestens mit 2-fach molarem Überschuss der Substanz II. Vorzugsweise wird dabei die wäßrige alkalische Suspension von vernetztem Polysaccharid in warmes Toluol gegossen und unter Rühren mit der Substanz II umgesetzt. Anschließend werden vorzugsweise die positiv geladenen

basischen Gruppen durch physiologisch verträgliche Anionen neutralisiert.

Als vernetzte Polysaccharide kommen vorzugsweise die bekannten vernetzten Polydextrane in Frage. Prinzipiell können aber auch andere quellbare und vernetzte Polysaccharide als Träger der positiven basischen Gruppen eingesetzt werden.

In den nachfolgenden Beispielen sind die neuen Zellkulturmikroträger, Verfahren zu ihrer Herstellung und ihre Verwendung näher erläutert.

Beispiel 1

Herstellung der Mikroträger:

13 g trockene, vernetzte Dextranperlen (Polydex PL-50 oder Sephadex G 50) mit einer Wasseraufnahme von etwa 5 g/g und einer Korngröße von 80 bis 100 µm werden in destilliertem Wasser über Nacht gequollen. Nach Absaugen des überschüssigen Wassers werden die gequollenen Perlen mit 5,2 g einer 43 gewichtsprozentigen Natronlauge vermischt. Die so hergestellte alkalische Suspension von Dextrankügelchen wird in 120 g auf 50 °C vorgewärmtes Toluol gegossen. Unter kräftigem Rühren werden zu dieser Reaktionsmischung 8 ml Chlorethyl-diethyl-amin zugegeben. Nach 3 Stunden Reaktionszeit bei 50 °C werden weitere 10 ml Chlorethyldiethylamin zugetropft.

Nach weiteren 3 Stunden Rühren bei 50 °C ist die Reaktion beendet. Die umgesetzten Dextranperlen werden durch Filtration aus der Reaktionsmischung abgetrennt. Durch mehrmaliges Waschen mit Alkohol und destilliertem Wasser sowie durch Einstellen des pH-Wertes mit verdünnter Salzsäure auf 0,5 werden die Mikroträger gereinigt. Die Entquellung erfolgt anschließend durch wiederholtes Behandeln der Perlen mit Waschlösungen steigender Alkoholkonzentration. Die entquollenen Perlen werden über Nacht bei 80 °C getrocknet.

Nach dieser Methode hergestellte Mikroträger enthalten ungefähr 4,0% Stickstoff und eine Ladungskapazität von 2,86 meq/g Mikroträger bzw. 4,66 meq/g des trockenen, nicht behandelten Dextrans.

Die Austauscherkapazität der so hergestellten Mikroträger beträgt 2,4 meq/g Mikroträger bzw. 3,91 meq/g trockenen, nicht behandelten vernetzten Dextrans.

Zur Bestimmung der Austauscherkapazität werden 1,0 g getrocknetem Mikroträger in destilliertem Wasser gequollen, in eine kleine Säule überführt und mit 0,1 n Salzsäure mehrfach gewaschen. Das Entfernen der nicht gebundenen Chloridionen erfolgt durch Spülen der Perlen mit $10^{-4}$n Salzsäure.

Durch Zugabe einer ausreichenden Menge von 10%-iger Natriumsulfatlösung werden die gebundenen Chloridionen verdrängt und durch Titration des Eluates mit 0,1 n Silbernitratlösung und Kaliumchromat als Indikator bestimmt.

Als Resultat erhält man dann meq $Cl^-$ pro Gramm Mikroträger. Die Umrechnung auf meq $Cl^-$ pro Gramm nicht behandelte Dextranperlen erfolgt nach folgender Formel:

Austauscherkapazität/g – trockenes, nicht behandeltes vernetztes Dextran =

$$1.000 \times \frac{\text{meq } Cl^-/\text{g Mikroträger}}{1.000 - 135 \times \dfrac{\%N \times 1.000}{100 \times 14}}$$

Die Bestimmung der Struktur der so gebundenen Stickstoffverbindungen erfolgt durch Titration von 1 g der trockenen Mikroträger in 20 ml einer 1 m Kaliumchloridlösung nach dem Einstellen des Ausgangs-pH-Wertes auf 12 mit 1,0 Salzsäure. Die Titrationskurve zeigt durch Abwesenheit einer Stufe im pH-Bereich von 10 bis 8,5, daß keine tertiären Aminogruppen mehr vorhanden sind. Der pKs-Wert der Aminoverbindung liegt zwischen 5 bis 7.

Beispiel 2

Durch Variation der Menge an eingesetzter Natronlauge und Chlorethyl-diethyl-amin werden die Mikroträger Nr. 434, 416, 432 und 435 hergestellt.

Beispiel 3

(Anwachskinetik von GMK-Zellen) (Green Monkey Kidney)

5 mg/ml Mikroträger, hergestellt gemäß Beispiel 1 bzw. 2 werden in bakteriologischen Petrischalen, 06 cm, 1 Stunde bei 37 °C mit 5 ml Medium MEM (Minimum Essential Medium) + 8% FBS (Fetal bovine serum) inkubiert.

Nach Zugabe von frischen, in Trypsin/Versen abgelösten GMK-Zellen, so daß sich eine Konzentration von $2{,}6 \times 10^5$ Zellen/ml Medium einstellte, erfolgt alle 15 Minuten eine Probenentnahme. Durch Auszählen der nichtgebundenen Zellen kann die Anwachsrate bestimmt werden.

Im Vergleich zu dem erfindungsgemäßen Zellträgermaterial wurde gleichzeitig die Anwachskinetik auch mit den auf dem Markt käuflich zu erwerbenden Mikrocarriern auf Dextranbasis (Cytodex®) mit einer Ladungsdichte von 2,0 meq/g neutrale Dextranmatrix und mit dem Anionenaustauscher Sephadex A-50® durchgeführt.

Die Resultate sind in Fig. 1 dargestellt und zeigen, daß die neuen Mikroträger auch bei hoher Ladungskapazität nicht toxisch sind, während sich mit Sephatex A-50 die bekannten toxischen Effekte nach etwa 75 Minuten zeigen.

Beispiel 4

Wachstum von verankerungsabhängigen Zellen am Beispiel von GMK-Zellen auf den Mikroträgern 416 und 421 mit unterschiedlicher Ladungsdichte.

Je 0,3 g der trockenen Mikroträger werden in 20 ml PBS gequollen und anschließend bei 115 °C 15 Minuten 15 psi sterilisiert.

Das PBS wird abgegossen und ersetzt durch warmes Kulturmedium. Als Kulturmedium dient z.B. MEM (Minimum Essential Medium) mit einem Zusatz von 8% fetal bovine serum (fötalem Kälberserum oder Kälberfötusserum). Die Perlsuspension wurde in einem Kulturgefäß, vorzugsweise eine Spezial-Spinnerflasche, überführt. Das Volumen wurde mit dem Kulturmedium + 8% FBS auf 100 ml aufgefüllt, begast und temperiert. Das Beimpfen erfolgte mit einem Zellinoculum von 1,3 × $10^7$ GMK-Zellen (= Green Monkey Kidney = Grüne Affennierenzellen), die aus Passage Nr. 127 des Institutes für Virologie der Universität Köln stammten und 5 Tage lang in Plastikflaschen mit Kulturmedium vorgezogen wurden.

Nach einer anfänglichen statischen Verankerungsphase wurde eine Rührgeschwindigkeit von 30 UpM eingestellt. Mediumwechsel erfolgte alle 48 Stunden. Das Zellwachstum wurde über einen Zeitraum von 170 Stunden hinweg überwacht, indem man die Zellen durch trypsinieren von den Carriern ablöste und nach einer modifizierten Sanfortet al-Methode (J. Natl. Cancer Inst. II, 737 (1951) zählte. Als Vergleich wurde gleichzeitig das Wachstum auf den bekannten Cytodex-1-Mikroträgern bestimmt. Das Ergebnis geht aus Fig. 2 hervor und zeigt, daß das Wachstum auf den neuen Mikroträgern auch bei hoher Ladungskapazität gut ist.

| Substanz | %N | A | B | C | D |
|---|---|---|---|---|---|
| 434 | 1,0 | 0,66 | 0,71 | 0,73 | 0,79 |
| 416 | 1,8 | 1,14 | 1,29 | 1,38 | 1,56 |
| 432 | 3,8 | 2,33 | 2,71 | 3,68 | 4,27 |
| 421 | 4,0 | 2,4 | 2,86 | 3,91 | 4,66 |
| 435 | 4,8 | 3,0 | 3,43 | 5,59 | 6,39 |
| Sephadex $A_{-50}$ | 4,2 | 2,7 | 3,0 | 4,54 | 5,04 |
| Cytodex-A | 1,8 | 1,2 | 1,29 | 1,45 | 1,56 |

A: Austauscherkapazität pro g Mikroträger

B: Ladungskapazität pro g Mikroträger

C: Austauscherkapazität pro g trockenes nichtbehandeltes vernetztes Dextran

D: Ladungskapazität pro g trockenes nicht behandeltes vernetztes Dextran

**Patentansprüche**

1. Zellkulturmikroträger mit positiv geladenen chemischen Anteilen aus vernetzten Polysacchariden und damit verknüpften basischen Gruppen, dadurch gekennzeichnet, daß der Zellkulturmikroträger nur mit den positiv geladenen, basischen «Tandem»-Gruppen mit einem pKs-Wert von 5–8 der Formel (I) substituiert ist

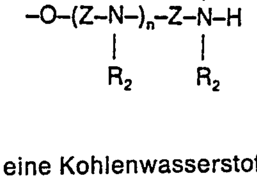

(I)

in welcher Z eine Kohlenwasserstoffkette aus 2–4 Kohlenstoffatomen ist, $R_1$ und $R_2$ gleich oder verschieden sind und Methyl-, Ethyl-, Propyl-, Isopropyl-, Butyl-, Phenyl-, Toluol- oder Benzylgruppen darstellen und n 1 bis 3 ist.

2. Zellkulturmikroträger gemäß Anspruch 1, dadurch gekennzeichnet, daß die positiv geladenen basischen Gruppen durch physiologisch verträgliche Anionen neutralisiert sind.

3. Verfahren zur Herstellung von Zellkulturmikroträgern mit positiv geladenen chemischen Anteilen aus vernetzten Polysacchariden und damit verknüpften basischen Gruppen durch Umsetzung des vernetzten Polysaccharids mit Substanzen der Formel (II)

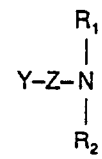

(II)

in der Z eine Kohlenwasserstoffkette aus 2–4 Kohlenstoffatomen ist, $R_1$ und $R_2$ gleich oder verschieden sind und Methyl-, Ethyl-, Propyl-, Isopropyl-, Butyl-, Phenyl-, Toluol- oder Benzylgruppen darstellen und Y eine reaktive Gruppe ist, dadurch gekennzeichnet, daß die Umsetzung in Gegenwart von starken Basen in mindestens zwei Stufen und mindestens mit 2-fach molarem Überschuß der Substanz II erfolgt.

4. Verfahren gemäß Anspruch 3, dadurch gekennzeichnet, daß die wäßrig alkalische Suspension von vernetztem Polysaccharid in warmes Toluol gegossen und unter Rühren mit der Substanz II umgesetzt wird.

5. Verfahren gemäß einem der Ansprüche 3 oder 4, dadurch gekennzeichnet, daß die positiv geladenen, basischen Gruppen anschließend durch physiologisch verträgliche Anionen neutralisiert werden.

6. Verwendung der Zellkulturmikroträger gemäß Ansprüchen 1 und 2 zur Züchtung von verankerungsabhängigen Zellen in einer Mikroträgerkultur.

**Claims**

1. A cell culture microcarrier having positively charged chemical portions comprising cross-linked polysaccharides and basic groups linked therewith, characterized in that the cell culture microcarrier is substituted only with positively charged basic "tandem" groups of a pKs value of from 5 to 8 represented by the formula (I)

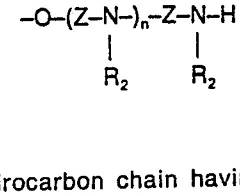

(I)

wherein

Z is a hydrocarbon chain having from 2 to 4 carbon atoms,

$R_1$ and $R_2$ are same or different and represent methyl, ethyl, propyl, isopropyl, butyl, phenyl, toluyl or benzyl groups and

n is 1 to 3.

2. The cell culture microcarrier according to claim 1, characterized in that the positively charged basic groups have been neutralized by physiologically compatible anions.

3. A method for preparing a cell culture microcarrier having positively charged chemical portions comprising cross-linked polysaccharides and basic groups linked therewith by reacting the cross-linked polysaccharide with a substance having the formula (II)

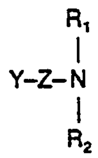 (II)

wherein

Z is a hydrocarbon chain having from 2 to 4 carbon atoms,

$R_1$ and $R_2$ are same or different and represent methyl, ethyl, propyl, isopropyl, butyl, phenyl, toluyl or benzyl groups and

Y is a reactive group,

characterized in that the reaction is carried out in the presence of strong bases in at least two steps and with use of at least two times the molar excess of the substance (II).

4. The process according to claim 3, characterized in that the aqueous alkaline suspension of cross-linked polysaccharide is poured into warm toluene and is reacted with substance (II) while stirred.

5. The process according to any one of claims 3 and 4, characterized in that the positively charged basic groups subsequently are neutralized by physiologically compatible anions.

6. Use of the cell culture microcarrier according to claims 1 and 2 for the cultivation of anchorage-dependent cells in a microcarrier culture.

## Revendications

1. Microsupport pour culture de cellules comportant des parties chimiques chargées positivement de polysaccharides réticulés et des groupes basiques qui y sont liés, caractérisé en ce que le microsupport de culture cellulaire n'est substitué qu'avec les groupes basiques «tandem» chargés positivement, d'une valeur de pKs de 5 à 8 de la formule (I)

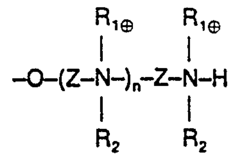 (I)

dans laquelle Z est une chaîne hydrocarbonée comportant 2 à 4 atomes de carbone, $R_1$ et $R_2$ sont identiques ou différents et représentent des groupes méthyle, éthyle, propyle, isopropyle, butyle, phényl, tolyle, ou benzyle et n est égal à 1 à 3.

2. Microsupport de culture cellulaire selon la revendication 1, caractérisé en ce que les groupes basiques chargés positivement sont neutralisés par des anions physiologiquement compatibles.

3. Procédé de fabrication de microsupport de culture cellulaire comportant des parties chimiques chargées positivement en polysaccharides réticulés et des groupes basiques qui y sont reliés par réaction du polysaccharide réticulé avec des substances de la formule (II)

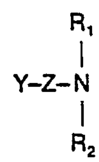 (II)

dans laquelle Z est une chaîne hydrocarbonée comportant 2 à 4 atomes de carbone, $R_1$ et $R_2$ sont identiques ou différents et représentent des groupes méthyle, éthyle, propyle, isopropyle, butyle, phényl, tolyle, ou benzyle et Y est un groupe réactif, caractérisé en ce que la réaction a lieu en présence de base forte en au moins deux étapes et avec un excédent molaire de la substance II au moins égal à deux fois.

4. Procédé selon la revendication 3, caractérisé en ce que la suspension alcaline aqueuse de polysaccharides réticulés est versée dans du toluène chaud et est mise à réagir avec la substance II sous agitation.

5. Procédé selon l'une des revendications 3 ou 4, caractérisé en ce que les groupes basiques, chargés positivement, sont ensuite neutralisés par des anions physiologiquement compatibles.

6. Utilisation du microsupport de culture cellulaire selon les revendications 1 et 2 pour la culture de cellules dépendantes d'une fixation d'ancrage dans une culture sur microsupport.

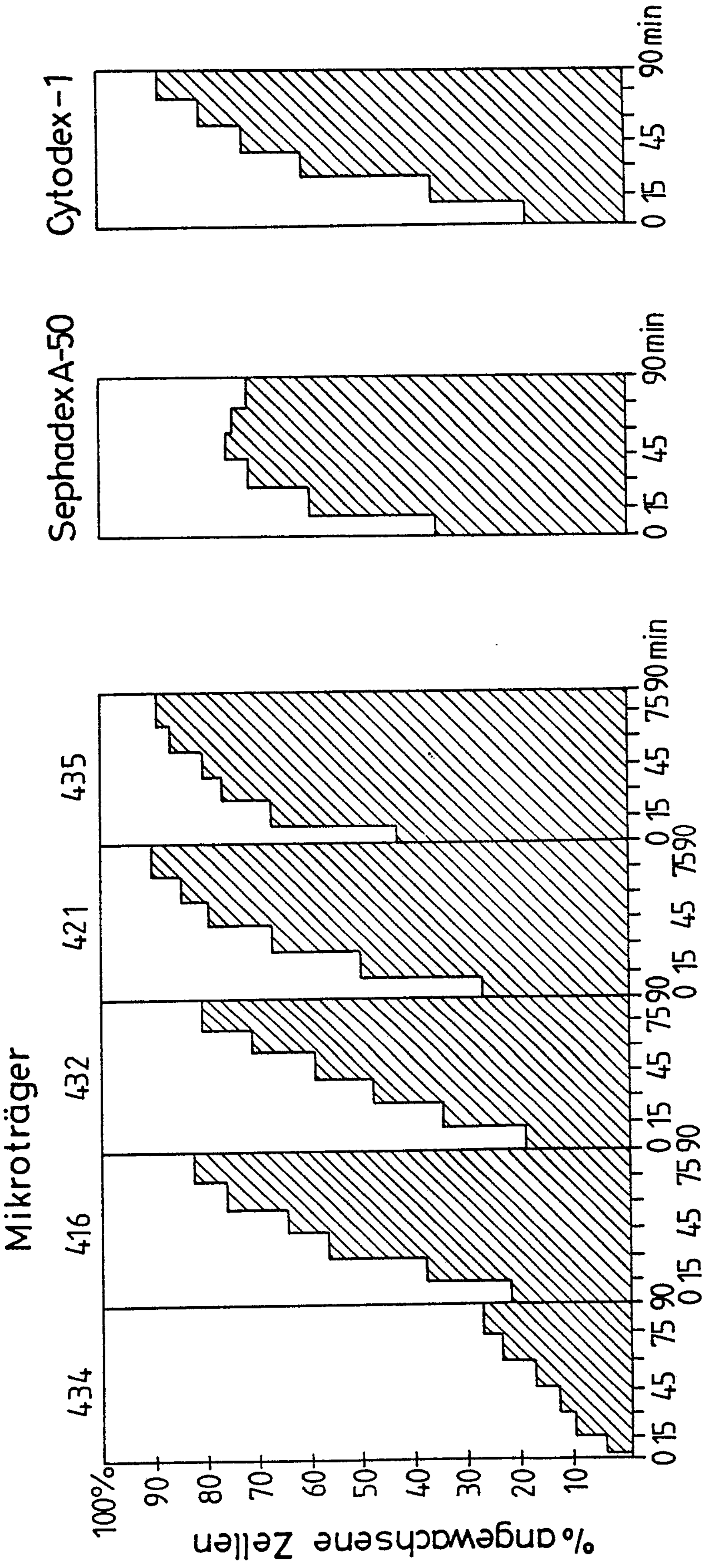


Anwachskinetik

FIG.1

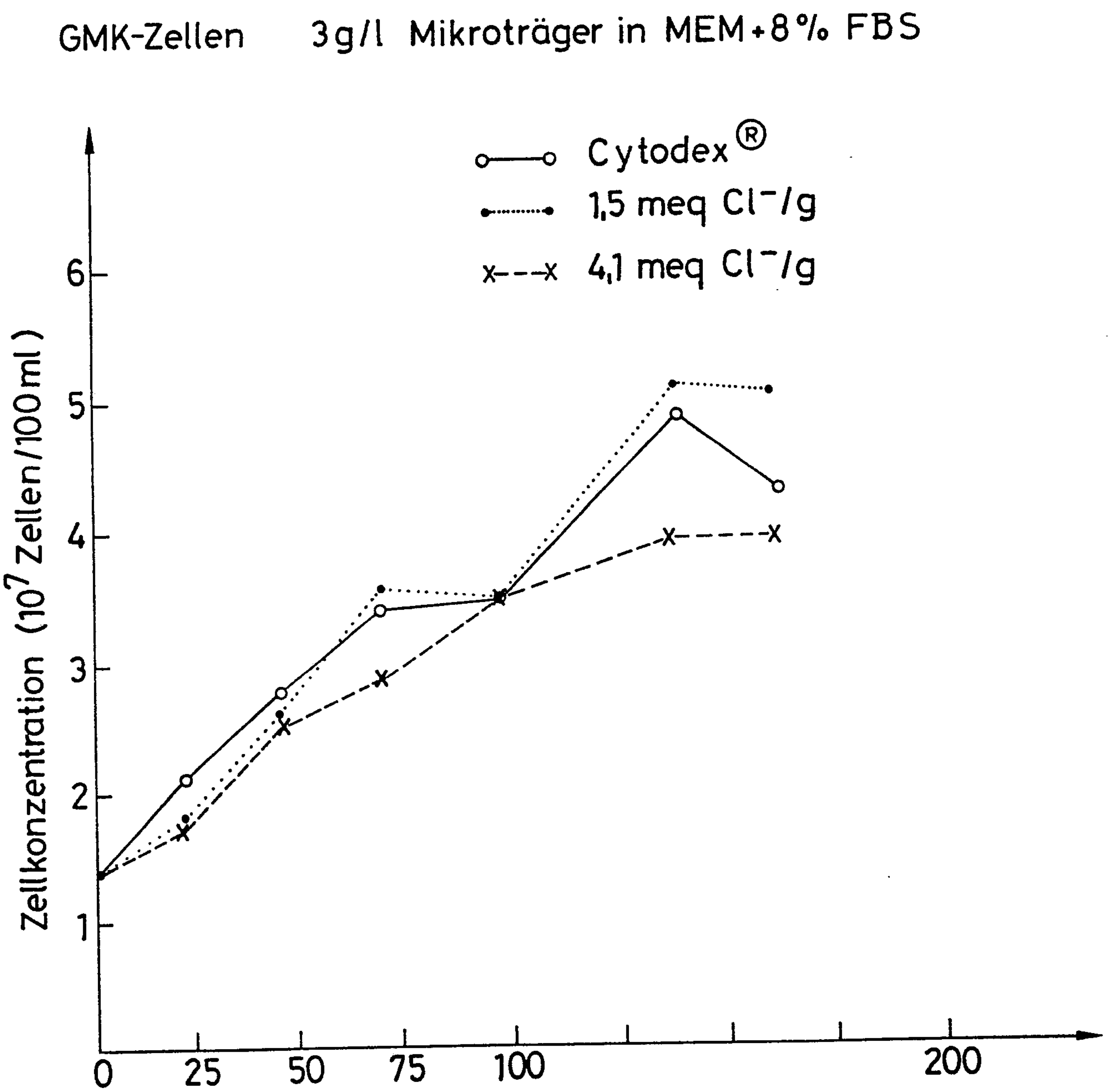


FIG.2 Zellvermehrungskultur